(19) Europäisches Patentamt
European Patent Office
Office européen des brevets

(11) EP 2 794 093 B1

(12) **EUROPEAN PATENT SPECIFICATION**

(45) Date of publication and mention
of the grant of the patent:
**06.11.2019   Bulletin 2019/45**

(51) Int Cl.:
*B01J 23/00* (2006.01)          *B01J 23/34* (2006.01)
*B01J 37/03* (2006.01)          *C07C 5/333* (2006.01)

(21) Application number: **12805595.1**

(86) International application number:
**PCT/EP2012/005211**

(22) Date of filing: **17.12.2012**

(87) International publication number:
**WO 2013/091822 (27.06.2013 Gazette 2013/26)**

(54) **ZINC AND MANGANESE ALUMINATE CATALYST USEFUL FOR ALKANE DEHYDROGENATION**

ZINK- UND MANGAN-ALUMINAT-KATALYSATOR ZUR VERWENDUNG BEI DER ALKAN-DEHYDRIERUNG

CATALYSEUR D'ALUMINATE DE MANGANÈSE ET ZINC UTILE POUR LA DÉSHYDROGÉNATION D'ALCANE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2011   EP 11010141**

(43) Date of publication of application:
**29.10.2014   Bulletin 2014/44**

(73) Proprietor: **Saudi Basic Industries Corporation Riyadh 11422 (SA)**

(72) Inventors:
• **SELVANATHAN, Anthonisamy**
  **Gujarat, Manjusar 391775 (IN)**
• **SUBHASH, Chandra Laha**
  **Gujarat, Manjusar 391775 (IN)**
• **VYAS, Maynak**
  **Gujarat, Manjusar 391775 (IN)**

(74) Representative: **Stiebe, Lars Magnus et al Balder**
  **Paseo de la Castellana 93, 5**
  **28046 Madrid (ES)**

(56) References cited:
  **EP-A1- 0 676 232        EP-A2- 0 557 982**
  **US-A- 5 344 805        US-A- 5 955 046**

• **A Maltha: "The Active Sites of Manganese- and Cobalt-Containing Catalysts in the Selective Gas Phase Reduction of Nitrobenzene", JOURNAL OF CATALYSIS., vol. 149, no. 2, 1 October 1994 (1994-10-01), pages 356-363, XP055256033, US ISSN: 0021-9517, DOI: 10.1006/jcat.1994.1303**

**EP 2 794 093 B1**

**Description**

The present invention relates to a catalyst composition suitable for the dehydrogenation of alkanes having 2-8 carbon atoms comprising zinc and manganese aluminate, optionally further comprising sodium (Na), potassium (K), caesium (Cs), rubidium (Rb), strontium (Sr), barium (Ba), magnesium (Mg), calcium (Ca), gallium (Ga), germanium (Ge),tin (Sn), copper (Cu), zirconium (Zr), cobalt (Co), tungsten (W) or mixtures thereof, wherein said catalyst composition preferably is essentially platinum free. Furthermore, a method for preparing said catalyst composition and a process for dehydrogenating alkanes having 2-8 carbon atoms, preferably isobutane, comprising contacting the said catalyst composition with said alkanes is provided.

Olefinic lower hydrocarbons such as propene, butenes and isobutene are very important intermediates in the petrochemical industry. Such olefins are primarily produced as co-products in catalytic and steam cracking processes. Alternatively, lower olefins can be commercially produced by catalytic dehydrogenation of the corresponding lower alkanes. US 3763255 for instance describes a method for dehydrogenation of C4-C30 hydrocarbons using a catalyst which comprises a platinum component, an iridium component and an alkali or alkaline earth metal component with a porous carrier material. The applicability of conventional endothermic dehydrogenation of lower alkanes, however, is limited by thermodynamic constraints and rapid catalyst deactivation caused by coke formation.

It has been previously described that zinc-aluminate based catalyst compositions are useful as catalysts in a process for dehydrogenation of lower alkanes. US 5,344,805; US 5,430,220; and EP 0 557 982 A2 describe a process for dehydrogenating at least one alkane comprising 2 to 8 carbon atoms to an alkene in the presence of steam and a catalyst composition comprising zinc aluminate, at least one tin oxide (i.e. SnO and/or $SnO_2$) and platinum. The zinc aluminate is prepared by a solid state method comprising calcining hydrated alumina and zinc oxide. Furthermore, EP 0 676 232 A1 discloses the preparation of spinel type $ZnAl_2O_4$ catalysts, which may comprise additional elements such as Mn, Sn and Ga for the reduction of nitrogen oxides and US 5, 955, 046 discloses the preparation of a spinel structure catalytic material comprising Ga, Zn and Al.

Finally, Maltha, A. et al., (1994), "The active sites of manganese- and cobalt-containing catalysts in the selective gas phase reduction of nitrobenzene", Journal of Catalysis, Vol. 149, no. 2, pp. 356-363, discloses the preparation of various manganese/zinc aluminate spinels of the general formula $MnxZn1-xAl_2O_4$ with $0<x<1$. These catalysts are suitable for the dehydrogenation of alkenes.

A major drawback of known zinc-aluminate based catalyst compositions useful as alkane dehydrogenation catalyst is that they require an additional metal like platinum as part of the catalyst composition to be effective. Without such an additional active metal the conversion of alkanes is greatly reduced. In addition thereto, it is described that the feed stream of a conventional dehydrogenation catalyst further comprises steam. It was an object of the present invention to provide a catalyst suitable for the dehydrogenation of alkanes with improved activity. Furthermore, it was an object of the present invention to provide an alkane dehydrogenation process which does not require steam to be present in the feed.

The solution to the above problem is achieved by providing the embodiments as described herein below and as characterized in the claims. Accordingly, the present invention provides a catalyst composition suitable for the dehydrogenation of alkanes having 2-8 carbon atoms comprising zinc and manganese aluminate, wherein the relative molar ratios of the elements comprised in said composition are represented by the formula

$$M/Zn_{1-y}Mn_yAl_2O_4$$

wherein:

0-5 wt-% M based on the zinc and/or manganese aluminate is present in the catalyst composition and M is selected from the group of sodium (Na), potassium (K), caesium (Cs), rubidium (Rb), strontium (Sr), barium (Ba), magnesium (Mg), calcium (Ca), gallium (Ga), germanium (Ge), or tin (Sn), copper (Cu), zirconium (Zr), cobalt (Co), tungsten (W) and mixtures thereof, wherein the zinc and/or manganese aluminate was calcined at 600-700°C, and y is in the range of $0<y<1$.

In the context of the present invention, it was surprisingly found that zinc and/or manganese aluminate comprising catalyst composition of the present invention provides a very high activity (as shown by a higher conversion and yield) and good selectivity for dehydrogenation of lower alkanes to alkenes. Preferably, the catalyst of the invention has a good selectivity towards isobutene. Furthermore, this high activity and/or selectivity may even be present in the absence of steam in the feed. Furthermore, the catalyst may have improved stability; that is it may maintain its activity for longer periods of use and/or more catalyst regeneration cycles.

Preferably, the catalyst composition of the present invention is essentially platinum free. As used herein, the term "essentially free" when relating to a certain (group of) element(s), preferably platinum, means to describe a catalyst composition wherein the comprised amount of said (group of) element(s) is too low to have an effect on catalyst performance. In one embodiment, the catalyst composition of the invention comprises less than 0.05 wt-% of said (group of) element(s), preferably less than 0.01 wt-% of said (group of) element(s), more preferably less than 0.005 wt-% said (group of) element(s) and even more preferably less than 0.001 wt-%. Particularly preferably, the content of said certain (group of) element(s) is below the detection limit of e.g. 60ppm for platinum, when using Atomic Adsorption Spectroscopy. Most preferably, the catalyst composition comprises no platinum. In one embodiment, the catalyst composition is essentially free from one or more elements selected from Group 10 of the Periodic Table (IUPAC version of 22 June 2007).

The zinc and manganese aluminate may have spinel structure. The term "spinel structure" is well known in the art and is defined herein as an aluminium comprising mixed oxide having the general formulation $Z^{2+}Al_2^{3+}O_4^{2-}$ which is crystallised in the cubic (isometric) crystal system and wherein the oxide anions are arranged in a cubic close-packed lattice and wherein the cations "Z" and Al occupy some or all of the octahedral and tetrahedral sites in the lattice.

The amount of zinc and manganese present in the zinc and manganese aluminate is determined by the molar ratio of zinc and manganese in relation to the aluminium. Accordingly, the molar ratio of zinc and manganese to aluminium ([Zn+Mn]:Al) in the zinc and manganese aluminate is 1:2 (also depicted as $Zn_{1-y}Mn_yAl_2$). The catalyst composition comprises zinc manganese aluminate, which is a mixed oxide comprising both zinc and manganese (also depicted as $Zn_{1-y}Mn_yAl_2$, wherein 0<y<1). More preferably, the molar ratio of zinc and manganese to aluminium in the zinc manganese aluminate (Zn-Mn-aluminate) is $Zn_{1-y}Mn_yAl_2$, wherein y is in the range of 0.01-0.99 (or "y=0.01-0.99"), even more preferably y=0.1-0.9 and most preferably y=0.4-0.6.

The zinc and manganese aluminate comprised in the catalyst composition of the invention may be modified with gallium (Ga) or tin (Sn). The amount of gallium or tin present in the modified zinc and manganese aluminate may be 0-5 wt-% gallium (Ga) or tin (Sn) based on the zinc and manganese aluminate. Preferably, the zinc and/or manganese aluminate comprises more than 0.001 wt-% Ga or Sn, even more preferably more than 0.01 wt-% Ga or Sn and most preferably more than 0.05 wt-% Ga or Sn based on the zinc and manganese aluminate. Preferably, the zinc and manganese aluminate comprises less than 1 wt-% Ga or Sn, even more preferably less than 0.5 wt-% Ga or Sn and most preferably less than 0.1 wt-% Ga or Sn based on the zinc and manganese aluminate.

For example, M may be gallium (Ga) or tin (Sn) in an amount of 0.01 - 0.1wt% based on the zinc and magnesium aluminate.

In a further aspect of the present invention a method for preparing a catalyst composition is provided. Accordingly, the present invention provides a method comprising the steps of

(a) preparing a solution of zinc- and manganese-comprising salts and of aluminium comprising salts to form a zinc- and manganese and aluminium-comprising solution;
(b) admixing a basic solution, preferably a sodium carbonate ($Na_2CO_3$) solution, to the zinc- and manganese and aluminium-comprising solution to form zinc and/or manganese aluminate; and
(c) calcining the zinc and manganese aluminate.

Preferably, the catalyst composition as defined herein above is prepared with the method for preparing a catalyst composition of the present invention.

In the solution preparation step (a), a solution of zinc- and manganese-comprising salts and of aluminium comprising salts is prepared to form a zinc- and manganese and aluminium-comprising solution. The solution may be made in any suitable solvent, preferably water, most preferably demineralised water. Suitable solvents are all liquid compounds in which the chosen salts are soluble and which are easy to remove when the solid catalyst particles are formed. The solvent and the obtained solution may be heated to at least 60 °C and up to 95 °C (60-95 °C), most preferably to 75-85 °C to facilitate dissolving of the zinc- and manganese-comprising salts and/or of the aluminium comprising salt. The preferred solvent is water, most preferably demineralised water.

Any source of zinc, manganese and aluminium that is soluble in the selected solvent may be used to prepare the zinc- and manganese and aluminium-comprising solution. Suitable zinc-, manganese- and aluminium- sources may be in the form of nitrate, chloride, carbonate, and bicarbonate. A particularly suitable soluble zinc salt is zinc nitrate hexahydrate, a particularly suitable soluble manganese salt is manganese (II) nitrate and a particularly suitable soluble aluminium salt is aluminium nitrate nonahydrate.

In the precipitation step (b) a basic solution, preferably sodium carbonate ($Na_2CO_3$) solution, is admixed to the zinc- and manganese and aluminium-comprising solution to form insoluble zinc and/or manganese aluminate, preferably under constant agitation. Other particularly suitable bases include, but are not limited to $K_2CO_3$, $(NH_4)_2CO_3$ and $NH_4OH$. Preferably, the base is added in a controlled fashion until the pH of the mixture reaches a value of 7.0-7.5. The temperature during the precipitation step may be kept at 60-95 °C, preferably at 75-85 °C. After adding the base the obtained mixture is preferably kept at elevated temperature under constant agitation for 0.5-5 hours.

After step (b) and before step (c) as described herein, the solid catalyst precursor (i.e. the solid phase of the mixture that is formed after completing the precipitation step (b)) is preferably separated from the liquid (i.e. the liquid phase of the mixture that is formed after completing the precipitate forming step (b)) using any conventional method which allows the separation of a precipitate from a liquid. Suitable methods include, but are not limited to, filtering, decanting and centrifugation. Subsequently the obtained solid may be washed, preferably using one of the solvents in which the solutions were made, more preferably with water, most preferably with distilled water. The solid then may be dried, preferably at 110-120 °C for 4-16 hours.

Finally in the calcination step (c), the catalyst precursor is calcined by heating the obtained zinc and manganese aluminate in an oxygen containing atmosphere. The catalyst precursor may be calcined at 600-700°C for 2-24 hrs.

A catalyst composition prepared using a calcination temperature of 600 - 700°C may be able to provide alkenes from alkanes with an even higher conversion and yield. Also, or alternatively, the catalyst composition prepared using a calcination temperature of 600-700°C may maintain its activity for a longer period of time.

The catalyst composition may then be contacted with a reducing agent after the calcining step (c) but prior to use, wherein said reducing agent preferably is selected from the group consisting of hydrogen ($H_2$) and hydrocarbons having 2 to 5 carbon atoms.

In one embodiment, a soluble M-comprising salt may be admixed to the zinc- and/ manganese and aluminium-comprising solution. The soluble M- comprising salt may be admixed before admixing the basic solution of in the precipitate forming step (b). Accordingly, the present invention provides a method comprising the steps of

(a) preparing a solution of zinc- and manganese-comprising salts and of aluminium comprising salts to form a zinc- and manganese and aluminium-comprising solution;
(b') admixing soluble M- comprising salt to form an M-modified zinc- and/or manganese and aluminium-comprising solution;
(b) admixing a basic solution, preferably a sodium carbonate ($Na_2CO_3$) solution, to the M-modified zinc- and manganese and aluminium-comprising solution to form M-modified zinc and manganese aluminate; and
(c) calcining the M-modified zinc and manganese aluminate.

For the avoidance of doubt, with an "M-comprising salt" is meant a salt of M, wherein M is selected from the group of sodium (Na), potassium (K), caesium (Cs), rubidium (Rb), strontium (Sr), barium (Ba), magnesium (Mg), calcium (Ca), gallium (Ga), germanium (Ge),tin (Sn), copper (Cu), zirconium (Zr), cobalt (Co), tungsten (W) and mixtures thereof.

Similarly, with zinc comprising salt, manganese comprising salt or aluminium comprising salt is meant a salt of zinc, respectively a salt of manganese respectively a salt of aluminium.

Any salt of zinc, manganese or aluminium that is soluble in the selected solvent may be used. For example, suitable salts may be in the form of nitrate, chloride, carbonate and bicarbonate. Preferably, one or more of the salts in the zinc comprising salt, the manganese comprising salt or the aluminium comprising salt is a nitrate salt.

Alternatively, the zinc and manganese aluminate formed after admixing the basic solution is contacted with an M-comprising salt solution to deposit the M selected from the group of sodium (Na), potassium (K), caesium (Cs), rubidium (Rb), strontium (Sr), barium (Ba), magnesium (Mg), calcium (Ca), gallium (Ga), germanium (Ge),tin (Sn), copper (Cu), zirconium (Zr), cobalt (Co), tungsten (W) and mixtures thereof on the zinc and/or manganese aluminate. Accordingly, the present invention provides a method comprising the steps of

(a) preparing a solution of zinc- and manganese-comprising salts and of aluminium comprising salts to form a zinc- and manganese and aluminium-comprising solution;
(b) admixing a basic solution, preferably a sodium carbonate ($Na_2CO_3$) solution, to the zinc- and manganese and aluminium-comprising solution to form zinc and/or manganese aluminate;
(b") contacting the formed zinc and manganese aluminate with a M-comprising salt solution to form the M-modified zinc and/or manganese aluminate; and
(c) calcining the M-modified zinc and manganese aluminate.

Any salt comprising M that is soluble in the selected solvent may be used to modify the zinc and manganese aluminate. Suitable salts may be in the form of nitrate, chloride, carbonate, and bicarbonate. For example, a particularly suitable soluble tin salt is tin chloride and a particularly suitable soluble gallium salt is gallium nitrate. Preferably, one or more of the salts in the M-comprising salt solution are nitrate salts. More preferably, one or more of the salts in the M-comprising salt solution, the zinc comprising salt, the manganese comprising salt or the aluminium comprising salt is a nitrate salt.

The invention therefore, also relates to a method for preparing the catalyst composition of the invention wherein the zinc- and manganese and aluminium-comprising solution further comprises M before admixing a solution of sodium carbonate ($Na_2CO_3$) in step (b), or wherein the zinc and manganese aluminate formed in step (b) is contacted with an M-comprising salt solution; wherein M in the M-comprising salt solution is selected from the group of sodium (Na), potassium (K), caesium (Cs), rubidium (Rb), strontium (Sr), barium (Ba), magnesium (Mg), calcium (Ca), gallium (Ga), germanium (Ge),tin (Sn), copper (Cu), zirconium (Zr), cobalt (Co), tungsten (W) and mixtures thereof.

The catalyst composition of the present invention is preferably formed in regularly sized particles such as conventionally formed catalyst pellets and/or sieved catalyst particles. The catalyst composition of the present invention may comprise further components such as diluents. Any inert catalyst diluent may be used. Preferably, the diluent is alpha alumina.

In a further embodiment of the present invention, a catalyst composition suitable for the dehydrogenation of alkanes having 2-8 carbon atoms comprising zinc and manganese aluminate is provided, wherein said catalyst composition is obtainable by the herein described method for preparing the catalyst composition. This catalyst composition is preferably essentially platinum-free. Accordingly, the present invention provides a catalyst composition obtainable by the method comprising the steps of

(a) preparing a solution of zinc- and manganese-comprising salts and of aluminium comprising salts to form a zinc- and manganese and aluminium-comprising solution;
(b) admixing a basic solution, preferably a sodium carbonate ($Na_2CO_3$) solution, to the zinc- and manganese and aluminium-comprising solution to form zinc and/or manganese aluminate; and
(c) calcining the zinc and manganese aluminate.

This catalyst composition can be readily distinguished from known zinc and/or manganese aluminate comprising catalysts by known methods such as by X-ray diffraction (XRD).

In a further embodiment of the present invention, a process for dehydrogenating alkanes having 2-8 carbon atoms is provided, wherein said process comprises contacting the catalyst composition as described herein with said alkanes.

It is evident for the skilled person that the process of the present invention is performed under alkane dehydrogenation conditions, preferably non-oxidative dehydrogenation conditions. Process conditions useful in the process of the present invention, also described herein as "alkane dehydrogenation conditions", can be easily determined by the person skilled in the art; see Horvath (2003) Encyclopaedia of Catalysis Volume 3, 49-79. Accordingly, the dehydrogenation process may be performed at a reaction temperature of 500-600 °C, a space velocity of 0.1-1 $h^{-1}$ and a pressure of 0.01-0.1 MPa.

The alkane having 2-8 carbon atoms preferably is propane or isobutane.

Accordingly, a process for dehydrogenating alkanes having 2-8 carbon atoms is provided comprising: preparing a catalyst composition comprising the steps of

(a) preparing a solution of zinc- and manganese-comprising salts and of aluminium comprising salts to form a zinc- and manganese and aluminium-comprising solution;
(b) admixing a basic solution, preferably a sodium carbonate ($Na_2CO_3$) solution, to the zinc- and manganese and aluminium-comprising solution to form zinc and manganese aluminate; and
(c) calcining the zinc and manganese aluminate; and
(d) contacting the catalyst composition with said alkanes under alkane dehydrogenation conditions.

Although the invention has been described in detail for purposes of illustration, it is understood that such detail is solely for that purpose and variations can be made therein by those skilled in the art without departing from the spirit and scope of the invention as defined in the claims.

It is further noted that the invention relates to all possible combinations of features described herein, preferred in particular are those combinations of features that are present in the claims.

It is further noted that the term 'comprising' does not exclude the presence of other elements. However, it is also to be understood that a description on a product comprising certain components also discloses a product consisting of these components. Similarly, it is also to be understood that a description on a process comprising certain steps also discloses a process consisting of these steps.

## Mode(s) for Carrying Out the Invention

The present invention will now be more fully described by the following non-limiting Examples.

## Brief description of the Figures

Figure 1 (Fig. 1) shows the powder XRD pattern of zinc aluminate as prepared in example 1.

Figure 2 (Fig. 2) shows the powder XRD pattern of zinc manganese aluminate as prepared in example 3.

Figure 3 (Fig. 3) shows the XRD pattern of the zinc aluminate prepared in example 7 as compared to the XRD pattern of zinc aluminate prepared by coprecipitation method.

Figure 4 (Fig. 4) shows the XRD pattern of the zinc manganese aluminate prepared in example 8.

Figure 5 (Fig. 5) shows the XRD pattern of zinc aluminate of example 1 (a) as compared to the zinc aluminate containing 0.05wt% Cs (0.05%Cs-ZnAl$_2$O$_4$) of example 9 (b).

Figure 6 (Fig. 6) shows the XRD profiles of the zinc aluminate catalysts of the invention containing different amounts of Copper (Cu).

**Example 1: Preparation of zinc aluminate.**

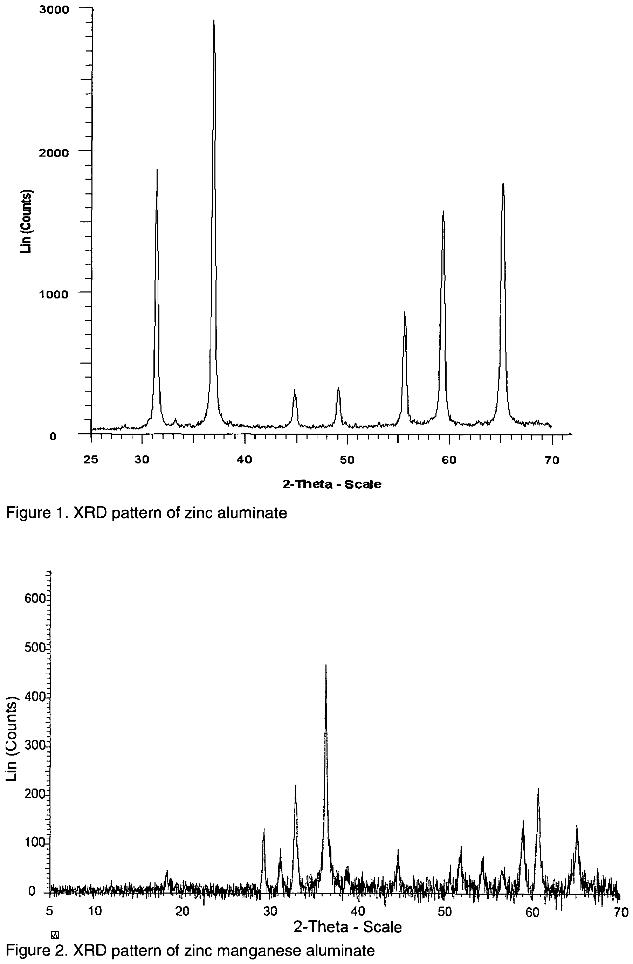

16.232 g zinc nitrate hexahydrate was dissolved in 60 ml demineralised water. 40.92 g of aluminium nitrate nonahydrate was dissolved in 110 ml of demineralised water. Both solutions were mixed in a 3-neck round bottom flask. 30 g of sodium carbonate was dissolved in 285 ml of demineralised water. The 3-neck flask containing the mixture of nitrate solutions was heated to 85°C with stirring. Then the sodium carbonate solution was added drop wise with continuous stirring. The addition was stopped when the pH of the hot mixture in the 3-neck flask was 7.0-7.5. This mixture was further digested at 100°C for 2.5 hours. Then the hot slurry formed was vacuum filtered and washed with demineralised water up to the pH of the filtrate was 7.0 and the sodium content in the filtrate was about 5 ppm. About 800 ml demineralised water was required for this washing. Then the wet cake was removed and dried at 120°C in an air oven for 12 hours. The dried solid was, then, calcined in a muffle furnace at 600°C for 4 hours in presence of air. The powder XRD pattern is given in Figure 1. X-ray diffraction data provided in the present application were obtained using a Bruker's D8 Advance system. Ni filtered Cu K$\alpha$ radiation (A=1.54056 A) was used to obtain X-ray diffraction (XRD) pattern. The X-ray source was operated at 40 kV and 30 mA and was scanned at a rate of 0.05 deg/min from 2θ value of 5° to 70°. The powdered samples were packed into a plastic slide for XRD data analysis.

**Example 2: Preparation of manganese aluminate.**

13.79 g of manganese (II) nitrate was dissolved in 80ml of demineralised water. 40.92 g of aluminium nitrate nonahydrate was dissolved in 110 ml of demineralised water. All three solutions were mixed in a 3-neck round bottom flask. 30 g of sodium carbonate was dissolved in 285 ml of demineralised water. The method of preparation was same as described in Example 1.

**Example 3: Preparation of zinc manganese aluminate.**

8.116 g zinc nitrate hexahydrate was dissolved in 30 ml demineralised water. 6.895 g of manganese (II) nitrate was dissolved in 40ml of demineralised water. 40.92 g of aluminium nitrate nonahydrate was dissolved in 110 ml of demineralised water. All three solutions were mixed in a 3-neck round bottom flask. 30 g of sodium carbonate was dissolved in 285 ml of demineralised water. The method of preparation was same as described in Example 1. The powder XRD pattern is given in Figure 2.

The catalysts of this invention and the other comparative catalysts were evaluated for lower alkane dehydrogenation reaction, particularly, isobutane dehydrogenation as follows: The catalyst powder and diluant (alpha alumina) powder were mixed thoroughly in the ratio 1:1. The mixture was pressed at 10 ton pressure to make pellets. The pellets were crushed and sieved to 0.5 to 1.0 mm size particles. 5 g of these particles were loaded in a down flow fixed bed micro catalytic reactor and pre-treated as follows:

Step 1: Exposed for 1 hr to air at the flow rate of 100 ml/min at 550°C
Step 2: Exposed for 10 min to nitrogen at the flow rate of 100 ml/min at 550°C
Step 3: Exposed for 1 hr to hydrogen at the flow rate of 100 ml/min at 550°C

After the pre-treatment, isobutane is fed to the reactor at 19 ml/min. The temperature of the catalyst bed before start of isobutane flow was maintained at 550°C. Pure isobutene was used as feedstream. The product stream coming out of the reactor was analyzed by an on-line Gas Chromatograph with a plot Al$_2$O$_3$/Na$_2$SO$_4$ column using a Flame Ionization Detector (FID). The isobutane conversion and isobutene selectivity were recorded. Table 1 presents the isobutane conversion and isobutene selectivity of some of the catalysts of this invention.

The provided values were calculated as follows:

Conversion:

An indication of the activity of the catalyst was determined by the extent of conversion of the isobutane. The basic equation used was:

$$\text{Conversion \%} = \text{Moles of isobutane}_{in} - \text{moles of isobutane}_{out}/\text{moles of isobutane}_{in} * 100/1$$

Selectivity:

First of all, the varying response of the detector to each product component was converted into %v/v by, multiplying them with online calibration factors. Then these were converted into moles by taking account the flow out of internal standard, moles of feed in and time in hours. Moles of each product were converted into mole-% and selectivity-% was measured by taking carbon numbers into account.

Yield:

The yield of a given process product can be calculated by multiplying the conversion with the fraction of selectivity.

**Table 1:** The isobutane conversion and isobutene selectivity on the catalysts at different time on stream

| Catalyst | Time on stream (min.) | Isobutane Conversion (%) | Isobutene Selectivity (%) |
|---|---|---|---|
| Zinc aluminate (Example 1) | 7 | 50.0 | 85.0 |
| | 41 | 41.3 | 91.4 |
| | 74 | 36.6 | 92.6 |
| | 106 | 34.6 | 93.6 |
| Manganese aluminate (Example 2) | 7 | 24.3 | 77.0 |
| | 41 | 19.9 | 75.3 |
| | 74 | 19.4 | 74.0 |
| | 106 | 18.7 | 72.9 |
| Zinc manganese aluminate (Example 3) | 8 | 48.9 | 92.5 |
| | 39 | 48.5 | 92.5 |
| | 71 | 49.1 | 92.5 |
| | 102 | 49.3 | 92.3 |

The catalyst of Example 3 (zinc manganese aluminate) is better than the catalysts of Example 1 (zinc aluminate) and Example 2 (manganese aluminate) with respect to conversion, selectivity and stability.

**Example 4: Preparation of Zn-Mn-aluminate with different compositions.**

Zinc manganese aluminate with compositions of zinc and manganese were prepared by the same method of Example 1 and varying the weight of zinc and manganese components. Both Zn and Mn ratios were varied 0.0-1.0 and the resulting compositions were as described in Table 2. The amount of aluminium nitrate for all catalysts was 40.92 g and the amount of sodium carbonate was 30 g.

**Table 2:** Zn and Mn ratios of catalyst compositions

| Catalyst | Structural formula | Zinc nitrate hexahydrate (g) | Aluminium nitrate nonahydrate (g) |
|---|---|---|---|
| 4.1 | $Zn_{0.9}Mn_{0.1}Al_2O_4$ | 14.609 | 1.379 |
| 4.2 | $Zn_{0.8}Mn_{0.2}Al_2O_4$ | 12.986 | 2.758 |
| 4.3 | $Zn_{0.7}Mn_{0.3}Al_2O_4$ | 11.362 | 4.137 |

(continued)

| Catalyst | Structural formula | Zinc nitrate hexahydrate (g) | Aluminium nitrate nonahydrate (g) |
|---|---|---|---|
| 4.4 | $Zn_{0.6}Mn_{0.4}Al_2O_4$ | 9.739 | 5.516 |
| 4.5 | $Zn_{0.4}Mn_{0.6}Al_2O_4$ | 6.493 | 8.274 |
| 4.6 | $Zn_{0.3}Mn_{0.7}Al_2O_4$ | 4.870 | 9.653 |
| 4.7 | $Zn_{0.2}Mn_{0.8}Al_2O_4$ | 3.246 | 11.032 |

The results of these catalysts for dehydrogenation of isobutane are given in Table 3. The dehydrogenation reaction was carried out by the procedure described in Example 3. The results show that Zn-Mn-aluminate with Zn=0.6-0.4 and Mn=0.4-0.6 (as indicated in the structural formula in Table 2) is the preferred composition range as these compositions are better than other compositions.

Table 3: Performance of zinc manganese catalysts with different compositions of Zn and Mn.

| Catalyst | Isobutane Conversion (%)* | Isobutene Selectivity (%)* |
|---|---|---|
| $Zn_{0.9}Mn_{0.1}Al_2O_4$ (Catalyst 4.1) | 51.4 | 83.6 |
| $Zn_{0.8}Mn_{0.2}Al_2O_4$ (Catalyst 4.1) | 50.9 | 86.4 |
| $Zn_{0.7}Mn_{0.3}Al_2O_4$ (Catalyst 4.1) | 47.6 | 91.0 |
| $Zn_{0.6}Mn_{0.4}Al_2O_4$ (Catalyst 4.1) | 49.9 | 89.6 |
| $Zn_{0.5}Mn_{0.5}Al_2O_4$ (Example 3) | 48.9 | 92.5 |
| $Zn_{0.4}Mn_{0.6}Al_2O_4$ (Catalyst 4.1) | 44.0 | 92.5 |
| $Zn_{0.3}Mn_{0.7}Al_2O_4$ (Catalyst 4.1) | 35.3 | 94.4 |
| $Zn_{0.2}Mn_{0.8}Al_2O_4$ (Catalyst 4.1) | 30.9 | 93.3 |
| *The conversion and selectivity given in this table are for reaction time of 8 min. | | |

**Example 5: Preparation of Zn-Mn-aluminate using different calcination temperatures.**

Zinc manganese aluminate catalysts were prepared by the same procedure described in Example 1 but calcined at different temperatures: 700°C, 800°C, 900°C and 1090°C.

The catalysts prepared by calcining at different temperatures were designated as follows:

Catalyst 5.1: Calcined at 700°C
Catalyst 5.2: Calcined at 800°C
Catalyst 5.3: Calcined at 900°C
Catalyst 5.4: Calcined at 1090°C

The results of these catalysts for dehydrogenation of isobutane are given in Table 4. The dehydrogenation reaction was carried out by the procedure described in Example 3. The conversion decreases with increase in temperature. The results show that the optimum temperature of calcination is 600-700°C.

Table 4: Performance of zinc manganese catalysts calcined at different temperatures.

| Calcination Temperature (°C) | Isobutane Conversion (%)* | Isobutene Selectivity (%)* |
|---|---|---|
| 600 | 49.1 | 92.5 |
| 700 | 47.7 | 93.1 |
| 800 | 21.1 | 91.6 |
| 900 | 18.5 | 85.0 |

(continued)

| Calcination Temperature (°C) | Isobutane Conversion (%)* | Isobutene Selectivity (%)* |
|---|---|---|
| 1090 | 12.1 | 60.1 |
| *The conversion and selectivity given in this table are for reaction time of 8 min. | | |

**Example 6: Preparation of Ga and Sn/Zn-Mn-aluminate.**

The Ga/Zn-Mn-aluminate catalysts with Ga 0.1 to 1.0 wt-% were prepared by the method described in Example 3 by adding required amount of gallium nitrate (0.0597 g for 0.1 wt-% Ga, 0.2976 g for 0.5 wt-% Ga and 0.5952 g for 1.0 wt-% Ga) also with other chemicals.

The Sn/Zn-Mn-aluminate catalysts were also prepared by method same as Ga/Zn-Mn-aluminate by taking required amounts of $SnCl_2$ (0.022 g for 0.1 wt-%, 0.095 g for 0.5 wt-% and 0.19 g for 1.0 wt-%). $SnCl_2$ was dissolved in water by adding about 2 ml of nitric acid. This solution was mixed with other nitrate salt solutions and the preparation was carried out as described above.

Both Ga/- and Sn/Zn-Mn-catalysts were evaluated for isobutane dehydrogenation by the procedure described in Example 3. The results are given in Table 5.

**Table 5:** Performance of Ga/- and Sn/Zn-Mn-aluminate catalysts for isobutane dehydrogenation.

| Catalyst | Isobutane Conversion (%) | Isobutene Selectivity (%) |
|---|---|---|
| Ga(0.1 wt-%)/ZnMnAl$_2$O$_4$ | 52.1 | 91.7 |
| Ga(0.5 wt-%)/ZnMnAl$_2$O$_4$ | 46.3 | 92.4 |
| Ga(1.0 wt-%)/ZnMnAl$_2$O$_4$ | 41.5 | 93.6 |
| Sn(0.1 wt-%/ZnMnAl$_2$O$_4$ | 47.2 | 94.5 |
| Sn(0.5 wt-%)/ZnMnAl$_2$O$_4$ | 41.3 | 94.6 |
| Sn(1.0 wt-%)/ZnMnAl$_2$O$_4$ | 37.5 | 94.4 |

Ga in the catalyst improves the conversion at lower concentration of Ga. The conversion decreases with increase of Ga above 0.1 wt-%. The presence of Sn improves the selectivity.

**Example 7: Preparation of Zn-aluminate by solid state method.**

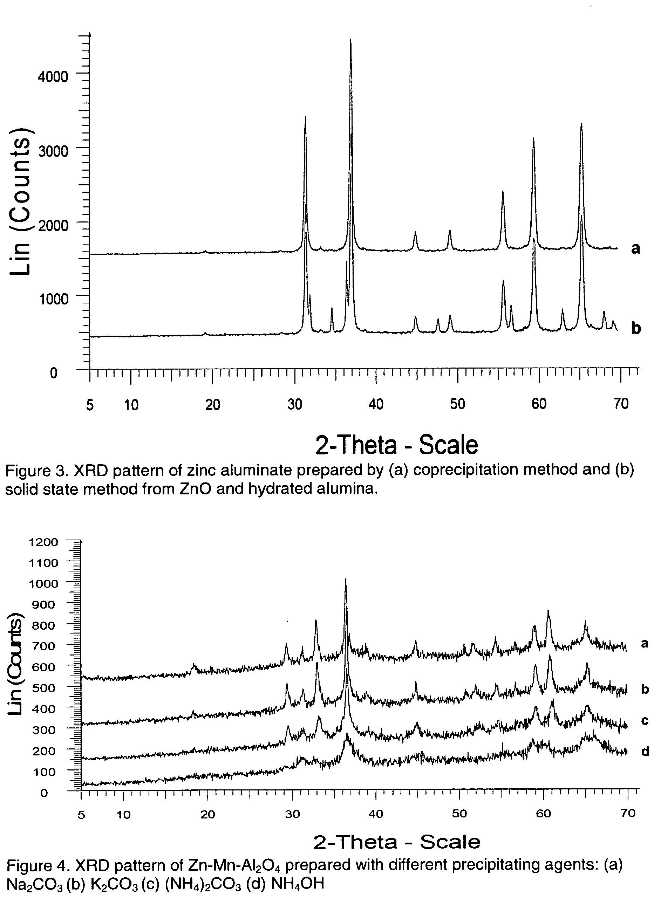

22.19 g of zinc oxide and 27.81 g of hydrated gamma-alumina are mixed thoroughly by grinding in a mortar with demineralised water to form a thick paste. The paste was dried at 120°C and calcined in air at 900°C for 8 hours. The XRD pattern (as obtained as described in Example 1) of this zinc aluminate is given in Figure 2 and XRD pattern of zinc aluminate prepared by coprecipitation method is also given Figure 3 for comparison. Accordingly, it is concluded that a different composition is obtained by preparing the Zn-aluminate with the co-precipitation method of the present invention than when using the solid state method of the prior art.

**Example 8: Preparation of Zn-Mn-aluminate with different precipitating agents.**

Zinc manganese catalysts were prepared by using different precipitating agents also. Potassium carbonate, ammonium carbonate and ammonium hydroxide were used as precipitating agents instead of sodium carbonate. The procedure is same as Example 3. These catalysts were evaluated for isobutane dehydrogenation by the procedure described in Example 3. The results are given in Table 6. The results show that the performance of these catalysts are less than the catalyst prepared using sodium carbonate as precipitating agent (Example 1). The XRD pattern of Zn-Mn-aluminate samples prepared with these precipitating agents are given in Figure4. The XRD pattern of Zn-Mn-aluminate prepared with sodium carbonate as precipitating agent is also given in Figure 4 for comparison. The XRD pattern was obtained as described above in example 1.

**Table 6:** Performance of zinc manganese catalysts prepared using different precipitating agents

| Precipitating Agent | Isobutane Conversion (%) | Isobutene Selectivity (%) |
|---|---|---|
| K$_2$CO$_3$ | 47.8 | 87.4 |

(continued)

| Precipitating Agent | Isobutane Conversion (%) | Isobutene Selectivity (%) |
|---|---|---|
| $(NH_4)_2CO_3$ | 41.4 | 89.6 |
| $NH_4OH$ | 35.6 | 93.2 |

**Example 9: Preparation of Zn-aluminate with different amounts of Cs.**

Previously prepared solutions of aluminium nitrate nonahydrate (40,9g in 54.4ml deionized water), zinc nitrate hexahydrate(16.2g in 27.3ml deionized water) and 7.33mg Caesium nitrate (for 0.05wt% Cs in the zinc aluminate catalyst) or 0.73mg Caesium nitrate (for 0.005wt% Cs in the zinc aluminate catalyst) and 100ml deionized water were transferred to a round-bottom flask, under stirring (250 rpm) and heated to 85°C. 1M sodium carbonate solution was added slowly to a pH of 8. The temperature was raised to 100°C and the precipitate was digested at 100°C for 2 hours. The contents were cooled, filtered and washed by hot air. The final pH of the washed liquid was 7. The wet cake was dried in an air oven at 120 for about 8 hours. The sample was powdered and calcinated at 900°C for 4 hours using a heating rate of 10°C per minute, an air flow rate of 150ml per minute. The weight of the final product was 9.1g.

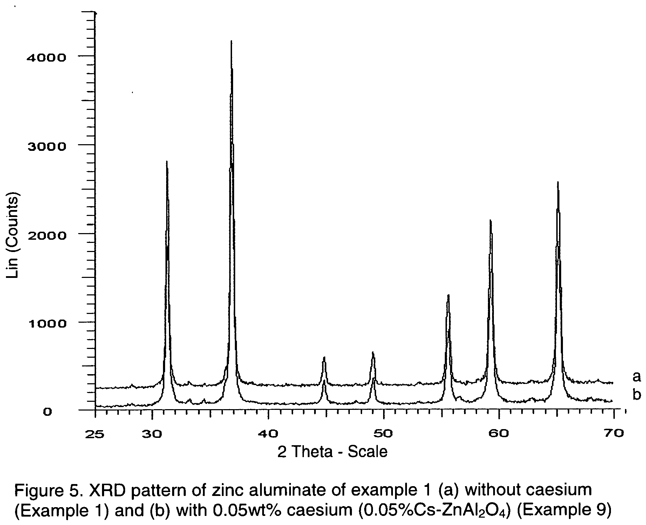

An XRD pattern was recorded. Figure 5 (Fig. 5) shows the XRD pattern of the zinc aluminate of example 1 (a) as compared to the zinc aluminate containing 0.05wt% Cs ($0.05\%Cs-ZnAl_2O_4$) (b).

A dehydrogenation reaction using these catalysts was carried out by the procedure described in Example 3. Conversion of isobutane and selectivity for isobutene were determined. The results are presented in Table 7.

Table 7: Performance of zinc/aluminate catalysts with different amounts of Cs.

| Catalyst | Conversion (%) | Selectivity (%) | Yield (%) |
|---|---|---|---|
| Cs (0wt%)/$ZnAl_2O_4$ | 44.0 | 96.0 | 42.2 |
| Cs (0.005wt%)/$ZnAl_2O_4$ | 46.8 | 95.6 | 44.7 |
| Cs (0.05wt%)/$ZnAl_2O_4$ | 50.3 | 94.9 | 47.7 |

As can be seen from Table 7, the Cs containing zinc-aluminate catalyst having 0.05wt%Cs based on the zinc-aluminate has better activity compared to the zinc-aluminate catalyst calcined at 900° C with increased conversion and yield.

Furthermore, it was found that these catalysts also maintain their activity for a longer period of operation.

**Example 10. Preparation of Zn-aluminate with different amounts of K.**

Analagous to example 9, a catalyst containing 0.05wt% K was prepared.

A dehydrogenation reaction using this catalyst and zinc aluminate catalyst 5 was carried out by the procedure described in Example 3.

The selectivity, conversion and yield were determined after 8 minutes. The results are presented in Table 8 below:

Table 8: Selectivity, conversion and yield of a 0.05wt% K containing zinc-aluminate catalyst as compared to a non-K containing zinc-aluminate catalyst after 40 min.

| | Conversion (%) | Selectivity (%) | Yield (%) |
|---|---|---|---|
| K (0wt%)/$ZnAl_2O_4$ | 44 | 96 | 42.2 |
| K (0.05wt%)/$ZnAl_2O_4$ | 49.3 | 95.5 | 47.1 |

As can be seen from Table 8, the presence of K in the zinc-aluminate catalyst of the invention improves conversion and yield in dehydrogenation of alkanes, while maintaining selectivity.

**Example 11. Preparation of Zn-aluminate with different amounts of Cu.**

Analagous to example 9, a zinc-aluminate catalyst was prepared containing different amounts of copper (Cu);

1wt% or 5wt% based on the zinc aluminate catalyst. Thereto, the following amounts of cupper nitrate trihydrate in deionized water were added to the zinc and aluminium nitrate solutions: 0.366 g copper nitrate trihydrate (for 1.0wt% Cu in the zinc aluminate catalyst), 1.867 g copper nitrate trihydrate (for 5.0wt% Cu in the zinc aluminate catalyst) and 3.731 g copper nitrate trihydrate (for 10.0wt% Cu in the zinc aluminate catalyst).

Calcination was performed either for 2 hours at 700°C (catalysts indicated with 700C) or for 4 hours at 900°C (catalysts indicated with 900C).

The Cu containing zinc-aluminate catalysts thus prepared were used in a dehydrogenation reaction using the procedure as described in example 3. The selectivity for isobutene, conversion and yield were determined.

The results are presented in Table 9 below.

**Table 9:** Performance of Cu containing zinc-aluminate catalysts.

| Catalyst | Conversion (%) | Selectivity (%) | Yield (%) |
|---|---|---|---|
| Cu (0wt%)/ZnAl$_2$O$_4$ 900C | 44.0 | 96.0 | 42.2 |
| Cu (1wt%)/ZnAl$_2$O$_4$, 700C | 56.4 | 80.1 | 45.2 |
| Cu (1wt%)/ZnAl$_2$O$_4$, 900C | 45.6 | 97.2 | 44.3 |
| Cu (5wt%)/ZnAl$_2$O$_4$, 900C | 45.2 | 97.5 | 44.1 |
| Cu (10wt%)/ZnAl$_2$O$_4$, 900C | 28.7 | 97.5 | 28.0 |

As can be seen from Table 9 above, calcination at 700°C for 2 hours leads to a catalyst that is more active for converting isobutane than the catalyst calcined at 900°C for 4 hours.

Furthermore, as shown, the optimal amount of M in the catalyst can easily be determined by the skilled person through routine experimentation.

Also, it is shown that the presence of M, in this case copper (Cu) up to 1wt% in the zinc aluminate catalyst of the invention increases the yield and conversion in an alkane dehydrogenation reaction using said catalyst.

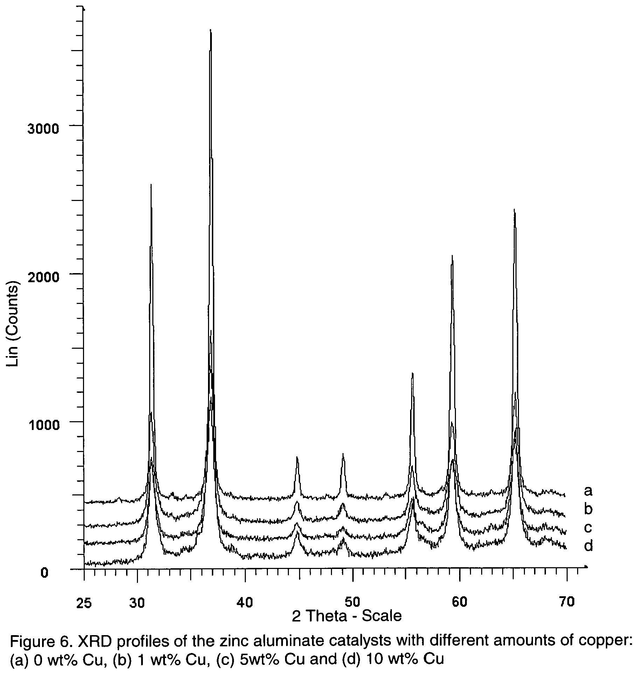

The XRD profiles of the zinc aluminate catalysts of the invention containing different amounts of Copper (Cu) were also recorded as described above and are given in Figure 6 (Fig. 6). In Fig. 6, (a) is the pure zinc aluminate catalyst; (b) is the zinc aluminate catalyst containing 1wt% Cu; (c)is the zinc aluminate catalyst containing 5wt% Cu; (d) is the zinc aluminate catalyst containing 10wt% Cu.

**Example 12. Preparation of Zn-aluminate with mixtures of M.**

Analogous to example 9, a zinc aluminate catalyst was prepared containing 0.05wt% of each of , K, Ca, Ba, Mg and Cs. Thereto, the following amounts of M were present in the zinc and aluminium containing solution: 12.930 mg potassium nitrate(for 0.05wt% K in the zinc aluminate catalyst), 29.461 mg calcium nitrate (for 0.05wt% Ca in the zinc aluminate catalyst), 9.515 mg barium nitrate (for 0.05wt% Ba in the zinc aluminate catalyst), 52.343 mg magnesium nitrate (for 0.05wt% Mg in the zinc aluminate catalyst) and 7.332 mg caesium nitrate (for 0.05wt% Cs in the zinc aluminate catalyst).

Also, a zinc aluminate catalyst was prepared containing 0.05wt%Cs and 1wt%Cu. Thereto, the following amounts of Cs and of Cu were present in the zinc and aluminium containing solution: 7.332 mg of caesium nitrate (for 0.05wt% Cs in the zinc aluminate catalyst) and 0.373 g copper nitrate trihydrate (for 1.0wt% Cu in the zinc aluminate catalyst).

Also, a zinc aluminate catalyst was prepared containing 1% zirconium (Zr), 0.05wt% Chromium (Cr) and 0.05wt% Potassium (K). Thereto, the following amounts of Zr, Cr and K were present in the zinc and aluminium containing solution: 0.253 g of zirconium nitrate (for 1.0wt% Zr in the zinc aluminate catalyst), 0.0385 g of chromium (III) nitrate nona hydrate (for 0.05wt% Cr in the zinc aluminate catalyst and 0.124 g potassium nitrate (for 0.05wt% K in the zinc aluminate catalyst).

The catalysts were used in a dehydrogenation reaction using the procedure as described in example 3 and selectivity, conversion and yield were determined.

The results are presented in Table 11 below.

**Table 10:** Performance of a zinc aluminate catalyst comprising a mixtures of M.

| Catalyst | Selectivity (%) | Conversion (%) | Yield (%) |
|---|---|---|---|
| Na, K, Ca, Mg and Cs (0.05wt% of each)/ZnAl$_2$O$_4$ | 95.9 | 50.3 | 48.3 |
| Cs (0.05wt%), Cu (1wt%)/ZnAl$_2$O$_4$ | 96.6 | 46.7 | 45.1 |

(continued)

| Catalyst | Selectivity (%) | Conversion (%) | Yield (%) |
|---|---|---|---|
| Zr (1wt%), Cr (0.05wt%), K (0.05wt%) /ZnAl$_2$O$_4$ 0.05wt%Cr | 95.3 | 50.0 | 47.6 |

As can be seen from Table 11, the zinc aluminate catalyst of the invention comprising a mixture of different M, shows a good conversion, selectivity and yield. Furthermore, it was found that the activity of the catalyst is maintained over longer periods of operation.

**Example 13. Preparation of zinc/aluminate catalysts with Zr.**

Zinc aluminate catalysts with Zr (0.5wt%, 1.0wt%; 5.0wt% or 8.0wt%) were prepared analogous to example 9. Thereto, the following amounts of or Zr were present in the zinc and aluminium containing solution: 0.127 g zirconium nitrate (for 0.5wt% Zr in the zinc aluminate catalyst), 0.254 g zirconium nitrate (for 1.0wt% Zr in the zinc aluminate catalyst), 1.267 g zirconium nitrate (for 5.0wt% Zr in the zinc aluminate catalyst) and 2.028 g zirconium nitrate (for 8.0wt% Zr in the zinc aluminate catalyst). Calcination was performed at 900°C for 4 hours.

The catalysts were used in a dehydrogenation reaction using the procedure as described in example 3 and selectivity, conversion and yield were determined.

The results are presented in Table 12 below.

**Table 11:** Performance of a zinc aluminate catalyst comprising Cr, Ce or Zr.

| Catalyst | Selectivity (%) | Conversion (%) | Yield (%) |
|---|---|---|---|
| ZnAl2O4 | 96.0 | 44.0 | 42.2 |
| | | | |
| Zr(0.5wt%)/ZnAl$_2$O$_4$ | 93.1 | 55.2 | 51.4 |
| Zr(1.0wt%)/ZnAl$_2$O$_4$ | 93.3 | 56.0 | 52.2 |
| Zr(5.0wt%)/ZnAl$_2$O$_4$ | 93.6 | 52.1 | 48.8 |
| Zr(8.0wt%)/ZnAl$_2$O$_4$ | 94.1 | 51.5 | 48.5 |

As can be seen from Table 12, a zinc aluminate catalyst of the invention comprising Zr in a non-oxidative dehydrogenation reaction of isobutane shows a good selectivity, in combination with high conversion and yield.

Furthermore, it was found that the activity of the catalyst could be maintained for longer periods of operation (more than 40 reaction-regeneration cycles).

**Claims**

1. Catalyst composition suitable for the dehydrogenation of alkanes having 2-8 carbon atoms comprising zinc and manganese aluminate, wherein the relative molar ratios of the elements comprised in said composition are represented by the formula

$$M/Zn_{1-y}Mn_yAl_2O_4$$

wherein:

0-5 wt-% M based on the zinc and manganese aluminate is present in the catalyst composition and M is selected from the group of sodium (Na), potassium (K), caesium (Cs), rubidium (Rb), strontium (Sr), barium (Ba), magnesium (Mg), calcium (Ca), gallium (Ga), germanium (Ge), or tin (Sn), copper (Cu), zirconium (Zr), cobalt (Co), tungsten (W) and mixtures thereof,
wherein the zinc and manganese aluminate was calcined at 600-700°C, and
y is in the range of $0<y<1$.

2. The catalyst composition according to claim 1, wherein said catalyst composition is essentially platinum free.

3. The catalyst composition according to claim 1 or 2, wherein the zinc and manganese aluminate has spinel structure.

4. The catalyst composition according to any one of claims 1-3, wherein y=0.01-0.99, preferably y=0.1-0.9, and most preferably y=0.4-0.6.

5. The catalyst composition according to any one of claims 1-4, wherein M is 0.01-0.1 wt-% gallium (Ga) or tin (Sn).

6. Method for preparing a catalyst composition as defined in any one of claims 1-5 comprising the steps of:

(a) preparing a solution of zinc- and manganese-comprising salts and of aluminium comprising salts to form a zinc- and manganese and aluminium-comprising solution,
(b) admixing a basic solution, preferably a sodium carbonate ($Na_2CO_3$) solution, to the zinc- and manganese and aluminium-comprising solution to form zinc and manganese aluminate, and
(c) calcining the zinc and manganese aluminate.

7. The method according to claim 6 wherein:

the zinc- and manganese and aluminium-comprising solution further comprises M before admixing a solution of sodium carbonate ($Na_2CO_3$) in step (b), or
wherein the zinc and manganese aluminate formed in step (b) is contacted with a M-comprising salt solution; wherein M in the M-comprising salt solution is selected from the group of sodium (Na), potassium (K), caesium (Cs), rubidium (Rb), strontium (Sr), barium (Ba), magnesium (Mg), calcium (Ca), gallium (Ga), germanium (Ge), tin (Sn), copper (Cu), zirconium (Zr), cobalt (Co), tungsten (W) and mixtures thereof.

8. The method according to claims 6 or 7, wherein one or more of the salts in the M-comprising salt solution are nitrate salts.

9. The method according to any one of claims 6-8, wherein the zinc and manganese aluminate is calcined for 2-24 hrs in an oxygen containing atmosphere, preferably air.

10. The method according to any one of claims 6-9, wherein the catalyst composition is contacted with a reducing agent after calcination, wherein said reducing agent preferably is selected from the group consisting of hydrogen ($H_2$) and hydrocarbons having 2 to 5 carbon atoms.

11. Process for dehydrogenating alkanes having 2-8 carbon atoms, preferably propane or isobutane, comprising contacting said alkanes with the catalyst composition according to any one of claims 1-5.

12. The process according to claim 11, wherein the process is performed at a reaction temperature of 500-600 °C, a space velocity of 0.1-1 h$^{-1}$ and a pressure of 0.01-0.1 MPa.

**Patentansprüche**

1. Katalysatorzusammensetzung, geeignet zur Dehydrierung von Alkanen mit 2-8 Kohlenstoffatomen, umfassend Zink- und Manganaluminat, wobei die relativen Molverhältnisse der in der genannten Zusammensetzung enthaltenen Elemente repräsentiert werden durch die Formel

$$M/Zn_{1-y}Mn_yAl_2O_4$$

wobei:

0-5 Gew.-% M, bezogen auf das Zink- und Manganaluminat, in der Katalysatorzusammensetzung vorhanden sind und M ausgewählt ist aus der Gruppe bestehend aus Natrium (Na), Kalium (K), Cäsium (Cs), Rubidium (Rb), Strontium (Sr), Barium (Ba), Magnesium (Mg), Calcium (Ca), Gallium (Ga), Germanium (Ge) oder Zinn (Sn), Kupfer (Cu), Zirkonium (Zr), Kobalt (Co), Wolfram (W) und Gemischen davon, wobei das Zink- und Manganaluminat bei 600-700 °C kalziniert wurde und y im Bereich von 0 < y < 1 liegt.

2. Katalysatorzusammensetzung nach Anspruch 1, wobei die Katalysatorzusammensetzung im Wesentlichen frei von Platin ist.

3. Katalysatorzusammensetzung nach Anspruch 1 oder 2, wobei das Zink- und Manganaluminat eine Spinellstruktur hat.

4. Katalysatorzusammensetzung nach einem der Ansprüche 1 - 3, wobei y = 0,01 - 0,99, vorzugsweise y = 0,1 - 0,9 und am bevorzugtesten y = 0,4 - 0,6 ist.

5. Katalysatorzusammensetzung nach einem der Ansprüche 1 - 4, wobei M 0,01 - 0,1 Gew.-% Gallium (Ga) oder Zinn (Sn) ist.

6. Verfahren zur Herstellung einer Katalysatorzusammensetzung wie in einem der Ansprüche 1 - 5 definiert, umfassend die folgenden Schritte:

(a) Herstellen einer Lösung von Zink und Mangan umfassenden Salzen und von Aluminium umfassenden Salzen, um eine Zink und Mangan und Aluminium umfassende Lösung zu bilden,
(b) Beimischen einer basischen Lösung, vorzugsweise einer Natriumcarbonat ($Na_2CO_3$) Lösung, zur Zink und Mangan und Aluminium umfassenden Lösung, um Zink- und Manganaluminat zu bilden, und
(c) Kalzinieren des Zink- und Manganaluminats.

7. Verfahren nach Anspruch 6, wobei:

die Zink und Mangan und Aluminium umfassende Lösung ferner M umfasst, bevor eine Lösung von Natrium-carbonat ($Na_2CO_3$) in Schritt (b) beigemischt wird, oder
wobei das in Schritt (b) gebildete Zink- und Manganaluminat mit einer M umfassenden Salzlösung in Kontakt gebracht wird;
wobei M in der M umfassenden Salzlösung ausgewählt ist aus der Gruppe aus Natrium (Na), Kalium (K), Cäsium (Cs), Rubidium (Rb), Strontium (Sr), Barium (Ba), Magnesium (Mg), Calcium (Ca), Gallium (Ga), Germanium (Ge), Zinn (Sn), Kupfer (Cu), Zirkonium (Zr), Kobalt (Co), Wolfram (W) und Gemischen davon.

8. Verfahren nach Anspruch 6 oder 7, wobei ein oder mehrere der Salze in der M umfassenden Salzlösung Nitratsalze sind.

9. Verfahren nach einem der Ansprüche 6 - 8, wobei das Zink- und Manganaluminat 2 - 24 Stunden lang in einer sauerstoffhaltigen Atmosphäre, vorzugsweise Luft, kalziniert wird.

10. Verfahren nach einem der Ansprüche 6 - 9, wobei die Katalysatorzusammensetzung nach dem Kalzinieren mit einem Reduktionsmittel in Kontakt gebracht wird, wobei das Reduktionsmittel vorzugsweise ausgewählt ist aus der Gruppe bestehend aus Wasserstoff ($H_2$) und Kohlenwasserstoffen mit 2 bis 5 Kohlenstoffatomen.

11. Prozess zur Dehydrierung von Alkanen mit 2 - 8 Kohlenstoffatomen, vorzugsweise Propan oder Isobutan, der das Inkontaktbringen der Alkane mit der Katalysatorzusammensetzung nach einem der Ansprüche 1 - 5 umfasst.

12. Prozess nach Anspruch 11, wobei der Prozess bei einer Reaktionstemperatur von 500 - 600 °C, einer Raumgeschwindigkeit von 0,1 - 1 $h^{-1}$ und einem Druck von 0,01 - 0,1 MPa durchgeführt wird.

**Revendications**

1. Composition de catalyseur appropriée pour la déshydrogénation d'alcanes ayant de 2 à 8 atomes de carbone comprenant un aluminate de zinc et de manganèse, dans laquelle les rapports molaires relatifs des éléments compris dans ladite composition sont représentés par la formule :

$$M/Zn_{1-y}Mn_yAl_2O_4$$

dans laquelle :

0 à 5 % en poids de M par rapport à l'aluminate de zinc et de manganèse sont présents dans la composition de catalyseur et M est choisi dans le groupe du sodium (Na), potassium (K), césium (Cs), rubidium (Rb), strontium (Sr), baryum (Ba), magnésium (Mg), calcium (Ca), gallium (Ga), germanium (Ge), ou étain (Sn), cuivre (Cu), zirconium (Zr), cobalt (Co), tungstène (W) et les mélanges de ceux-ci, dans laquelle l'aluminate de zinc et de manganèse a été calciné à 600 à 700 °C et y est dans la gamme de 0<y<1.

2. Composition de catalyseur selon la revendication 1, laquelle composition de catalyseur est essentiellement exempte de platine.

3. Composition de catalyseur selon la revendication 1 ou 2, dans laquelle l'aluminate de zinc et de manganèse a une structure de spinelle.

4. Composition de catalyseur selon l'une quelconque des revendications 1 à 3, dans laquelle y=0,01 à 0,99, de préférence y=0,1 à 0,9 et préférentiellement y=0,4 à 0,6.

5. Composition de catalyseur selon l'une quelconque des revendications 1 à 4, dans laquelle M est de 0,01 à 0,1 % en poids de gallium (Ga) ou d'étain (Sn).

6. Procédé de préparation d'une composition de catalyseur telle que définie selon l'une quelconque des revendications 1 à 5, comprenant les étapes consistant :

(a) à préparer une solution de sels comprenant du zinc et du manganèse et de sels comprenant de l'aluminium pour former une solution comprenant du zinc et du manganèse et de l'aluminium,
(b) à mélanger une solution basique, de préférence une solution de carbonate de sodium ($Na_2CO_3$), avec la solution comprenant du zinc et du manganèse et de l'aluminium pour former un aluminate de zinc et de manganèse, et
(c) à calciner l'aluminate de zinc et de manganèse.

7. Procédé selon la revendication 6, dans lequel :

la solution comprenant du zinc et du manganèse et de l'aluminium comprend en outre M avant de mélanger une solution de carbonate de sodium ($Na_2CO_3$) dans l'étape (b), ou
dans lequel l'aluminate de zinc et de manganèse formé dans l'étape (b) est mis en contact avec une solution de sel comprenant M ;
dans lequel M dans la solution de sel comprenant M est choisi dans le groupe du sodium (Na), potassium (K), césium (Cs), rubidium (Rb), strontium (Sr), baryum (Ba), magnésium (Mg), calcium (Ca), gallium (Ga), germanium (Ge), étain (Sn), cuivre (Cu), zirconium (Zr), cobalt (Co), tungstène (W) et les mélanges de ceux-ci.

8. Procédé selon la revendication 6 ou 7, dans lequel un ou plusieurs des sels dans la solution de sel comprenant M sont des sels de nitrate.

9. Procédé selon l'une quelconque des revendications 6 à 8, dans lequel l'aluminate de zinc et de manganèse est calciné pendant 2 à 24 heures dans une atmosphère contenant de l'oxygène, de préférence de l'air.

10. Procédé selon l'une quelconque des revendications 6 à 9, dans lequel la composition de catalyseur est mise en contact avec un agent réducteur après calcination, dans lequel ledit agent réducteur est de préférence choisi dans le groupe constitué par l'hydrogène ($H_2$) et les hydrocarbures ayant de 2 à 5 atomes de carbone.

11. Procédé de déshydrogénation d'alcanes ayant de 2 à 8 atomes de carbone, de préférence le propane ou l'isobutane, comprenant la mise en contact desdits alcanes avec la composition de catalyseur selon l'une quelconque des revendications 1 à 5.

12. Procédé selon la revendication 11, lequel procédé est mis en œuvre à une température de réaction de 500 à 600 °C, une vitesse spatiale de 0,1 à 1 $h^{-1}$ et une pression de 0,01 à 0,1 MPa.

Figure 1. XRD pattern of zinc aluminate

Figure 2. XRD pattern of zinc manganese aluminate

Figure 3. XRD pattern of zinc aluminate prepared by (a) coprecipitation method and (b) solid state method from ZnO and hydrated alumina.

Figure 4. XRD pattern of Zn-Mn-Al$_2$O$_4$ prepared with different precipitating agents: (a) Na$_2$CO$_3$ (b) K$_2$CO$_3$ (c) (NH$_4$)$_2$CO$_3$ (d) NH$_4$OH

Figure 5. XRD pattern of zinc aluminate of example 1 (a) without caesium (Example 1) and (b) with 0.05wt% caesium (0.05%Cs-ZnAl$_2$O$_4$) (Example 9)

Figure 6. XRD profiles of the zinc aluminate catalysts with different amounts of copper: (a) 0 wt% Cu, (b) 1 wt% Cu, (c) 5wt% Cu and (d) 10 wt% Cu

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3763255 A
- US 5344805 A
- US 5430220 A
- EP 0557982 A2
- EP 0676232 A1
- US 5955046 A

### Non-patent literature cited in the description

- **MALTHA, A. et al.** The active sites of manganese- and cobalt-containing catalysts in the selective gas phase reduction of nitrobenzene. *Journal of Catalysis,* 1994, vol. 149 (2), 356-363
- **HORVATH.** Encyclopaedia of Catalysis. 2003, vol. 3, 49-79